# EUROPEAN PATENT APPLICATION

(11) **EP 3 308 704 A1**
(43) Date of publication of application: **18.04.2018**
(21) Application number: 16806937.5
(22) Date of filing: 09.06.2016
(51) Int. Cl.: A61B 5/053

(54) **DEVICE FOR MEASURING DIFFERENCES IN CONDUCTIVITY**

(30) Priority: 09.06.2015 ES 201530807; 07.06.2016 ES 201630768
(71) Applicant: Dental Catch Iberica, S.L., 50012 Zaragoza (ES)
(72) Inventor: MAIRAL NEVOT, Mariano, 50012 Zaragoza (ES)
(74) Representative: Schäfer, Matthias W.
(86) International application number: PCT/ES2016/070435
(87) International publication number: WO 2016/198717

(57) **Abstract**

Device for measuring differences in conductivity that allows early detection of infectious/inflammatory periodental and/or apical diseases or in joint and/or muscle regions, before they can even be detected radiologically, the said device comprising an electrical emitter (1) in millivolts, with a given negative-peak square wave and a given frequency, both defined by a pulse width and interval between pulses, which, by means of two electrodes, one stationary (2) and one movable (3) is able to:
• Produce a mild tingling sensation on the patient's skin immediately over the teeth apices or joint and/or muscle regions as well as dermatological, glandular and integumentary areas affected by inflammation and/or infection.
• Display the intensity of the condition affecting the tissue to the professional on an illuminated scale display (15).

## Description

### Technical field

This invention relates to the sector of auxiliary means to aid diagnosis for detecting and locating inflammatory and infectious processes in different medical specialties such as traumatology, where it detects small foci in intra-articular and periarticular sites or muscle-tendon sites; in dermatology and especially in the field of dentistry and stomatology.

The basic object of the invention is the development of device for measuring differences in conductivity that allows early detection of infectious/inflammatory periodental and/or apical diseases, before they can even be detected radiologically. The device comprises a millivolt emitter, with a given negative-peak square wave and a given frequency, both defined by a pulse width and interval between pulses, by means of two electrodes, which can:
- Produce a mild tingling sensation in the patient's skin immediately above dental apices or joint and/or muscle regions affected by inflammation and/or infection.
- Display the intensity of the condition affecting the tissue to the professional on an illuminated scale display.

### Background of the invention

The existence is known of devices for locating and examining dental tissue, in particular, for the examination of apices, caries, bacterial infection, plaque, calculus and tartar. Thus, invention patent ES2177445 describes a gutta-percha apex locator point for dental use, which is provided, either internally or laterally, with an electrode in biocompatible material that ends at a minimal distance from the end of the said point.

Patent EP2260761 describes a dental device for examining optical characteristics of dental tissue, in particular, to examine caries, with means for generating excitation radiation directed onto the tooth examined, means of detection and means of evaluation to detect and evaluate a response radiation arising from the irradiated tooth tissue region in response to the radiation and a means of display.

Patent JP2001299699 describes a device to locate caries, bacterial infection, etc., with means for generating a light beam guided onto the tooth or tissue examined, with means of detection and evaluation to detect fluorescence in the irradiated tissue region.

Another solution is presented in patent WO2013040074, which describes a hand-held device for locating the apex of a root canal that includes an apex locator electrode, extending from a distal end of the housing for contacting an electrically conductive endodontic instrument positioned within a root canal during use.

Another example is presented in patent US20090148810, which describes a wireless dental apex locator for determining its location, which comprises an electronic module, having a battery power source, an impedance analyzer circuit and a radio frequency transmitter, including a grounding module with a connection clip and a module with an endodontic probe.

Patent WO2012144913 describes a device for detecting secondary caries, which comprises an emitter or generator to produce asinewave within a certain range of frequencies chosen from among values of200 Hz to 100 kHz and an amplitude of 50 mV to 5 V, connected to two electrodes, one stationary and the other movable, also having a control unit provided with a keyboard and a display unit and with components for measuring stimuli, also with connection to the two electrodes. Voltage in millivolts and frequency are adjustable. For this device to work, both electrodes must be placed inside the oral cavity, one on the mucous membrane and the other on the tooth itself, which may be uncomfortable for the patient; furthermore the cells emit very different responses if the wave is a sine signal wave, or if it is a negative-peak square wave, since cells are more sensitive to the latter.

Another example is presented in patent EP1384449, which describes an apical foramen locator to determine its location in relation to a tool inserted into the patient's tooth, by means of detecting impedance carried out via two electrodes and a stimulus voltage which can be modified to include a 100 mV peak-to-peak sine wave at 30 KHz

This device, as in the previously described case, operates with the electrodes in the oral cavity, generating a sine wave.

Patent WO9742909 describes an apparatus for the electronic detection of dental caries which comprises at least one probe electrode placed in electrical contact with the surface of the patient's tooth and a second electrode placed in electrical contact with another part of the body of the patient, being able to incorporate different types of probes to adapt to different types of teeth or different teeth surfaces, comprising an insulating substrate of hydrophobic material, such as Goretex, and one or more electrodes, preferably of a conductive, hydrophobic material such as carbon-impregnated Goretex.

Another example is described in patent US8326413, which comprises an apparatus for the detection of dental caries on the surface of a tooth, present underneath a crown, which involves the use of an electrically conductive pathway from the crown exterior to the tooth itself. The objective of the invention is that the dentist may undertake proper therapeutic measures to treat such teeth, in order to avoid far more expensive repairs such as the implementation of bridges or implants.

In all of the examples described for locating and inspecting apices, caries, bacterial infection, etc., the devices act directly on the teeth, mucous membranes or the areas of tissue affected, it being desirable to be able to examine such areas without having to act directly on them, for example, on the face skin in the areas corresponding to the teeth apices that are to be examined.

In this type of device the generalised use of electrical sine waves is also common, it being desirable to use a square wave with peak, which generates a more accurate and higher quality response from cells, in comparison to sine waves.

Other types of devices of a similar configuration are also known, intended for the detection of possible tumours in breast tissue; for example patent US20120065539 describes a device with a signal generator with one or a plurality of source electrodes attached where the breast joins with the chest wall, a receiving electrode attached to the nipple of the same breast; a processor or computer program which computes the spectrum of frequencies and correlates magnitudes and phases with given algebraic conditions, a computer or processor program which compares the signature results of healthy tissue and non-healthy tissue to extract the pattern to determine the characteristics of the mass, selected from the group consisting of presence, location, and type of abnormality, and a display or indicators to show the results to the user.

### Description of the invention

In order to improve the aforementioned drawbacks as far as possible, a device for measuring differences in conductivity has been devised, of the the type that comprises two electrodes, one stationary and one movable, which enable early detection of infectious/inflammatory periodental and/or apical diseases, or in joint and/or muscle regions, before they can even be detected radiologically, the said device comprising:
- An alphanumeric display with excitation button (T1) for the pulse width, repolarization button (T2) for the interval between pulses +/- progress buttons to increase or reduce voltage (V1), according to the mode of use,
- An electrical millivolt emitter with electrical negative-peak square wave and specific frequencies defined by parameters and voltages, connected to two electrodes, one stationary with a disposable adhesive patch at its free end and one movable, with a blunt tip, interchangeable and sterilisable, that may be a multipurpose movable electrode.
- An electrical stimulus gauge with three connections for the aforementioned stationary electrode, movable electrode and multipurpose movable electrode.
- illuminated scale display, with scale adjustment buttons,
- Connection to mains electricity via a medical grade battery charger with indicator light and on/off button
- Device start/stop button, all contained in a casing.

If the movable electrode is of the multipurpose type, it includes another illuminated scale in the electrode holder so that quantification of the biological response can be viewed on the device and on the electrode at the same time.

The alphanumeric display includes two utilities:
- show the level of excitation or type of stimulation and
- measure the response numerically

The excitation button for pulse width (T1) and the repolarization button, for the interval between pulses (T2) include an indicator light to indicate their activation.

The +/- progress buttons are configured with four buttons, two that indicate a scale of negative values -1 and -5 and two buttons that indicate positive values +1 and +5.

The electrical emitter, with negative-peak square wave, is configured with the following parameters and voltages:
- pulse width (T1): adjustable from 1 ms to 6 ms, by pressing the excitation button cyclically, indicating values of +1
- interval between pulses (T2): adjustable from 80 ms to 220 ms, by pressing the repolarization button cyclically, indicating values in intervals of 40 ms.,
- Voltage (V1): adjustable from 0 to 10 V., by means of the +/- progress buttons, indicating values in percentages of between 1% and 5%
   - adjusted by default to 25% of 10V, for infectious/inflammatory periodontal and/or apical diseases
   - adjustable to 40% of 10V for infectious/inflammatory diseases in joint and/or muscle regions.

The negative-peak square wave generates a more accurate and higher quality response from cells, in comparison to the sine waves that are generally used in this type of device.

With the patient comfortably seated, the stationary electrode is placed on the patient's forearm, in an area without hair. If the patient has a lot of hair then a zone without hair needs to be found or a zone has to be shaved to ensure perfect contact with the bare skin as it is attached using a disposable adhesive patch.

The movable electrode with blunt tip, interchangeable and sterilizable, either multipurpose or not, is applied to the face skin, that must be clean and without cream. It is advisable to move it in an orderly way from quadrant to quadrant and apply it, without pressing too hard, to each portion of the corresponding skin, over the teeth apices or the joint and/or muscle regions to be studied; the technology of the device is based on measuring the differences in conductivity between healthy tissue and inflamed or infected tissues and differentiating the level of infection.

By means of the stationary/movable or stationary/multipurpose movable electrodes, the electrical negative-peak square waves, adjusted via the excitation and repolarization buttons, cause a tingling sensation on the patient's skin just over teeth apices or joint and/or muscle regions affected by inflammation and/or infection, indicating to the professional, via the illuminated scale display and, where appropriate, via the illuminated scale on the multipurpose movable electrode, the degree to which the tissue is affected, given that minimal changes in tissue homeostatis produce changes in the electrical conductivity of the said tissue.

To avoid false positives and false negatives when carrying out examinations with the device disclosed, the device has excitation and repolarization buttons in order for the professional to be able to adjust the pulse width and the interval between pulse, regulating the output voltage via the +/- progress buttons, depending on the greater or lesser sensitivity of the person to be examined.

Highly sensitive patients may find the stimulus sensation to be excessive while other people may not manifest any type of sensation with the same signal intensity.

If, during the initial examination, the patient finds the signal to be excessive, its intensity should be lowered until the stimulus is bearable but not null. Likewise, if no type of signal is perceived, its strength can be increased.

The connection for a movable electrode and the connection for a multipurpose movable electrode are configured with the same colour, while the connection for a stationary electrode incorporates an activation indicator light, configured with a different colour to avoid connection errors.

The stationary/movable or stationary/multipurpose movable electrode combination is connected to the electrical emitter with the electrical stimulus gauge.

The illuminated scale display provides intuitive viewing of the biological response to the stimulus, adjusted via the scale buttons, configured one with an upward arrow and the other with a downward arrow, in the following way:

With the scale button, represented by an upward arrow, the number of leds indicating the biological response to the electrical stimulus can be multiplied, so that the number of leds that light up, with the same signal intensity, can be magnified by 2, 4 or 8.

The magnification required can be obtained by pressing the scale buttons, shown as an upward arrow or a downward arrow. This representation makes it easier to read the signal and quantify the biological response.

Connection to mains electricity via a medical-grade battery charger is shown by an indicator light providing visual information on the battery charge status which is recorded on the alphanumeric display.

The ON/OFF button acts in the following way
- in ON position to start the battery charging process,
- in OFF position to start the process in which the device operates without connection to the mains.

The contents of the priority document are deemed to be included in their entirety and by reference, in the present application.

### Advantages of the invention

The device for measuring differences in conductivity that is presented affords numerous advantages over the devices currently available, the most important of these being the early detection of infectious/inflammatory periodental and/or apical diseases, or in joint and/or muscle regions, even before they can be detected radiologically, through the combination of a stationary electrode and a movable electrode or of a stationary electrode and a multipurpose movable electrode in connection with an electrical emitter, with electrical negative-peak square wave, generating a cell response of greater quality and accuracy.

Another important advantage deriving from the previous one is that of incorporating a multipurpose movable electrode, which includes the illuminated scale in the electrode holder itself, allowing quantification of the biological response to be viewed on the device and on the electrode at the same time.

An added advantage is that the connection of the stationary electrode is configured with a different colour to that configured on the connections of the movable electrodes, to avoid connection errors.

A further advantage is that the technology of the device disclosed is based on measuring the differences in conductivity between healthy tissue and inflamed or infected tissue and on differentiating the level of infection via the electrodes applied exclusively on the patient's skin.

Another important advantage is that the device for measuring differences in conductivity clearly shows the alteration in the tissue both for the patient and the doctor so that both of them can see the need to establish treatment.

Another added advantage deriving from the previous one is that the doctor can adjust the intensity and type of electrical signal depending on the patient's sensitivity, to eliminate both false positives and false negatives, allowing detection of the specific positive signal.

And finally, it can also be added as an advantage that the device for measuring differences in conductivity is involved in locating and evidencing focal inflammatory processes and infections in different medical specialties such as traumatology, where it detects small foci at intra-articular or periarticular sites or muscle-tendon sites and in dermatology.

A person skilled in the art will easily comprehend that the characteristics of different embodiments can be combined with the characteristics of other possible embodiments, provided that the combination is technically possible.

### Description of the figures

To gain a better understanding of the object of this invention, a preferred practical embodiment is shown in the drawing attached.
Figure - 1 - shows a plan view of the device for measuring differences in conductivity.
Figure - 2 - schematically shows the electrical negative-peak square wavelength.

### Preferred embodiment of the invention

The conformation and characteristics of the invention can be better understood in the following description that relates to the attached figures.

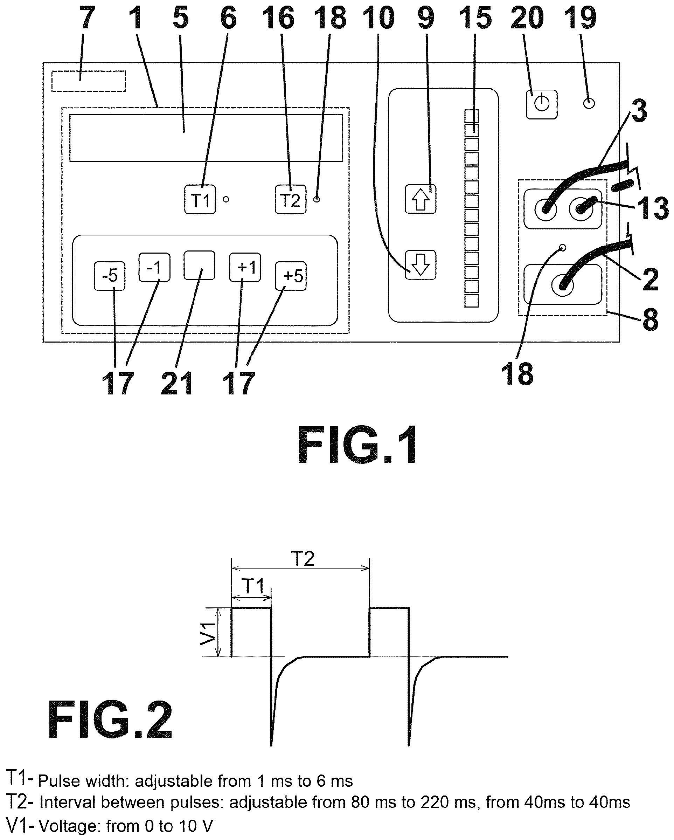

As can be seen in figure 1, the device for measuring differences in conductivity is presented, showing the casing (4), an alphanumeric display (5) with excitation button (6) for the pulse width (T1), repolarization button (16) for the interval between pulses (T2), with activation indicator light (18) and +/- progress buttons (17) to increase or reduce the voltage (V1), the alphanumeric display (5) incorporating the mode of use:
- by default, adjusted to 25% of 10V, for infectious/inflammatory periodontal and/or apical diseases
- adjusted manually to 40% of 10V for infectious/inflammatory diseases in joint and/or muscle regions.

It also shows a illuminated scale display (15) to make it easier to read the signal and to quantify biological response, adjusted by means of scale buttons (9 and 10).

It indicates a connection for movable electrode (3) and next to it, a connection for a multipurpose movable electrode (13), both connections being configured with the same colour.

Below these connectors, a connector for a stationary electrode (2) is shown, configured with a different colour to avoid connection errors, and an activation indicator light (18) is also shown.

Application of the stationary electrode (2) is always carried out on the patient's skin, on an area without hair or that is shaved to obtain perfect contact with the bare skin as it is attached by means of a disposable adhesive patch.

The movable electrode (3) with blunt tip, interchangeable and sterilizable, is applied to the skin of the face that is clean and without cream; it is applied to the skin surface, without exerting excessive pressure, on each portion of the corresponding skin over the teeth apices to be examined.

The multipurpose movable electrode (13) has the same application and includes the illuminated scale in the electrode holder itself so that the quantification of biological response can be viewed on the device and on the electrode at the same time.

Electrodes 2-3 or 2-13 are connected to an electrical emitter (1) and to an electrical stimulus gauge (8)
On the casing (4) there is a connection (7) to mains electricity via a medical-grade battery charger, with an indicator light (19) showing the battery charge status, which is recorded graphically on the alphanumeric display (5) on a charge bar.

On the top part of the device the ON/OFF button (20) is indicated
- in the ON position to start the battery charging process,
- in the OFF position to start the process of operation without connection to the mains.

The start/stop button (21) is also indicated in the position between the +/- progress buttons (17)

Figure 2 shows the diagram and table of the negative-peak square wave and frequencies, defined by:
- pulse width (T1): adjustable from 1 ms to 6 ms, by pressing the excitation button (6) cyclically, indicating values of +1
- interval between pulses (T2): adjustable from 80 ms to 220 ms, by pressing the repolarization button (16) cyclically, indicating values in intervals of 40 ms.,
- Voltage (V1): adjustable from 0 to 10 V., by means of the +/- progress buttons (17), indicating values in percentages of between 1% and 5%
   - adjusted by default to 25% of 10V, for infectious/inflammatory periodontal and/or apical diseases
   - adjustable to 40% of 10V for infectious/inflammatory diseases in joint and/or muscle regions.

The contents of the priority document are deemed to be included in their entirety and by reference, in the present application.

## Claims

1. Device for measuring differences in conductivity, of the type comprising one stationary electrode (2) and one movable electrode (3), **characterised in that** it comprises:
• An alphanumeric display (5) with excitation button (6) for the pulse width (T1), repolarization button (16) for the interval between pulses (T2), +/- progress buttons (17) to increase or reduce the voltage (V1) parameters, according to the mode of use.
• An electrical millivolt emitter with electrical negative-peak square wave and specific frequencies defined by parameters and voltages, connected to two electrodes, one stationary (2) with a disposable adhesive patch at its free end and one movable (3), with blunt tip, interchangeable and sterilizable, that may be a multipurpose type movable electrode (13).
• An electrical stimulus gauge (8) with three connections for the aforementioned stationary (2), movable (3) and multipurpose movable (13) electrodes.
• An illuminated scale display (15), with scale adjustment buttons (9) and (10),
• Connection to mains electricity (7) via a medical-grade battery charger with indicator light (19) and on/off button (20)
• Start/stop button (21) of the device, and all contained in a casing (4).

2. Device for measuring differences in conductivity, according to the preceding claim, **wherein** the electrical emitter (1) is configured with the following parameters and voltages:
• pulse width (T1): adjustable from 1 ms to 6 ms, by pressing the excitation button (6) cyclically, indicating values of +1
• interval between pulses (T2): adjustable from 80 ms to 220 ms, by pressing the repolarization button (16) cyclically, indicating values in intervals of 40 ms,
• Voltage (V1): adjustable from 0 to 10 V., by means of the +/- progress buttons (17), indicating values in percentages of between 1% and 5%
• adjusted by default to 25% of 10V, for infectious/inflammatory periodontal and/or apical diseases
• adjustable to 40% of 10V for infectious/inflammatory diseases in joint and/or muscle regions.

3. Device for measuring differences in conductivity, according to the preceding claims, **wherein** the +/- progress buttons (17) are configured with four buttons, two that indicate a scale of negative values -1 and -5 and two buttons that indicate positive values +1 and +5.

4. Device for measuring differences in conductivity, according to the preceding claims , **wherein** the upward scale button (9) multiplies the number of leds that indicate the biological response to the electrical stimulus, magnifying by 2,4 or 8 the number of leds that light up with the same signal intensity, adjusting magnification via the downward scale button (10).

5. Device for measuring differences in conductivity, according to the preceding claims, **wherein** the connection for a movable electrode and the connection for a multipurpose movable electrode are configured with the same colour, while the connection for a stationary electrode is configured with a different colour, avoiding connection errors.

6. Device for measuring differences in conductivity, according to the previous claims, **wherein** the multipurpose movable electrode (13), includes the illuminated scale in the electrode holder itself.
